(19) Europäisches Patentamt

European Patent Office

Office européen des brevets

(11) Veröffentlichungsnummer: **0 461 541 B1**

(12) **EUROPÄISCHE PATENTSCHRIFT**

(45) Veröffentlichungstag der Patentschrift: **14.12.94**

(21) Anmeldenummer: **91109202.1**

(22) Anmeldetag: **05.06.91**

(51) Int. Cl.5: **C07C 51/493**

(54) Verfahren zur Racematspaltung von 2,2-Dimethylcyclopropancarbonsäure.

(30) Priorität: **14.06.90 CH 1999/90**

(43) Veröffentlichungstag der Anmeldung:
**18.12.91 Patentblatt 91/51**

(45) Bekanntmachung des Hinweises auf die
Patenterteilung:
**14.12.94 Patentblatt 94/50**

(84) Benannte Vertragsstaaten:
**AT BE CH DE DK ES FR GB GR IT LI LU NL SE**

(56) Entgegenhaltungen:
**EP-A- 0 022 972**

(73) Patentinhaber: **LONZA AG**
**Gampel/Wallis**
**Münchenerstrasse 38**
**CH-4002 Basel (CH)**

(72) Erfinder: **Meul, Thomas, Dr.**
**Meisenweg 1**
**Visp (Kanton Wallis) (CH)**

(74) Vertreter: **Weinhold, Peter, Dr. et al**
**Patentanwälte Dipl.-Ing. G. Dannenberg**
**Dr. P. Weinhold**
**Dr. D. Gudel**
**Dipl.-Ing. S. Schubert**
**Dr. P. Barz**
**Siegfriedstrasse 8**
**D-80803 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung ist ein Verfahren zur Herstellung optisch reiner 2,2-Dimethylcyclopropancarbonsäure durch Racematspaltung.

2,2-Dimethylcyclopropancarbonsäure ist ein wichtiges Zwischenprodukt, beziehungsweise für die Synthese des Enzyminhibitors Cilastatin (EP 0 048 301) und von Insektiziden des Pyrethrintyps (GB-PS 1 260 847).

Insbesondere für die Herstellung von pharmazeutischen Wirkstoffen ist es wünschenswert, 2,2-Dimethylcyclopropancarbonsäure in optisch reiner Form, d.h. in Form des reinen (S)-(+)- oder des reinen (R)-(-)-Enantiomeren zur Verfügung zu haben. Da die chemische Synthese vom 2,2-Dimethylcyclopropancarbonsäure die Verbindung in Form ihres Racemats liefert, ist es erforderlich, eine Spaltung dieses Racemats durchzuführen. Solche Racematspaltungen werden üblicherweise dadurch bewirkt, dass man zunächst mittels einer optisch aktiven Hilfssubstanz das zu trennende Enantiomerengemisch in ein Gemisch diastereomerer Derivate überführt, welches sich aufgrund der unterschiedlichen physikalischen Eigenschaften von Diastereomeren durch fraktionierende Kristallisation oder Chromatographie trennen lässt. Aus den so getrennten Diastereomeren wird dann im Idealfall jeweils ein reines Enantiomeres der zu trennenden Verbindung und die optisch aktive Hilfssubstanz in Freiheit gesetzt.

In der Realität gelingt mit einer gegebenen Hilfssubstanz, auch wenn diese optisch völlig rein ist, meistens nur die unvollständige Abtrennung eines reinen Enantiomeren, so dass ein Gemisch zurückbleibt, welches überwiegend aus dem anderen Enantiomeren besteht. In weniger günstigen Fällen kann keines der beiden Enantiomeren in reiner Form isoliert werden.

Als Derivate von Carbonsäuren zu Zweck der Racematspaltung werden häufig deren Salze mit optisch aktiven Basen, insbesondere Aminen, verwendet. Diese Salze haben den Vorteil, dass sie sich sehr leicht und schnell bilden und durch Zusatz einer starken Säure auch wieder spalten lassen. Zur Racematspaltung von 2,2-Dimethylcyclopropancarbonsäure wurden bereits (S)-(-)-1-Phenylethylamin (GB-PS 1 260 847), (-)-N-Methylephedrin (JP-Anm. 60 56 936 und 60 56 942), Chinin (EP-Anm. 0 161 546) und verschiedene 1,2-Diphenylethylamine (EP-Anm. 0 093 511) eingesetzt.

Mit 1-Phenylethylamin konnte weder eine befriedigende Ausbeute noch eine ausreichende optische Reinheit erreicht werden.

Chinin lieferte ein Enantiomeres in guter optischer Reinheit, aber schlechter Ausbeute, für N-Methylephedrin wurde keine Ausbeute angegeben.

Bei 1,2-Diphenylethylamin ist die Ausbeute befriedigend und die optische Reinheit sehr gut, das Reagens ist jedoch, wie auch N-Methylephedrin, sehr teuer.

Weiterhin ist bekannt, dass 2,2-Dimethylpropancarbonsäure über die diastereomeren Menthylester, welche aus dem Säurechlorid mit (+)- bzw. (-)-Menthol erhältlich sind, in die Enantiomeren getrennt werden kann (US-PS 4 487 956). Dieses Verfahren liefert zwar brauchbare Ausbeuten und optische Reinheiten, ist jedoch relativ umständlich bei der Aufarbeitung und benötigt das relativ teure Menthol.

Aufgabe der vorliegenden Erfindung war daher, ein Verfahren zur Racematspaltung von 2,2-Dimethylcyclopropancarbonsäure zur Verfügung zu stellen, welches einfach durchzuführen ist und nur preiswerte und möglichst ungiftige optisch aktive Hilfssubstanzen benötigt.

Erfindungsgemäss wird diese Aufgabe durch das Verfahren nach Patentanspruch 1 gelöst.

Überraschend wurde gefunden, dass optisch aktiver Mandelsäuremethylester nicht nur mit (RS)-2,2-Dimethylcyclopropancarbonsäurechlorid an der OH-Gruppe glatt zu den entsprechenden diastereomeren Estern reagiert, sondern dass letztere auch durch fraktionierende Kristallisation trennbar sind und nach ihrer Hydrolyse die freigesetzte 2,2-Dimethylcyclopropancarbonsäure sehr leicht von der ebenfalls entstehenden Mandelsäure abgetrennt werden kann. Mandelsäure ist nämlich im Gegensatz zur 2,2-Dimethylcyclopropancarbonsäure in Alkanen praktisch unlöslich und bleibt bei der Extraktion mit diesen Lösungsmitteln in der wässrigen Phase zurück.

Das erfindungsgemässe Verfahren wird vorteilhaft solcherart durchgeführt, dass die racemische 2,2-Dimethylcyclopropancarbonsäure zunächst in das entsprechende Säurechlorid übergeführt wird. Dieser Schritt ist an sich bekannt und kann beispielsweise mit Thionylchlorid in Gegenwart katalytischer Mengen N,N-Dimethylformamid durchgeführt werden. Das so erhaltene Säurechlorid wird vorteilhaft durch Destillation gereinigt.

Anschliessend wird das racemische Säurechlorid mit optisch aktivem Mandelsäuremethylester unter Zusatz einer Hilfsbase zur Bindung des entstehenden Chlorwasserstoffs umgesetzt. Als Hilfsbase wird beispielsweise Pyridin eingesetzt. Die Veresterung wird vorteilhaft in einem inerten Lösungsmittel wie beispielsweise Dichlormethan durchgeführt.

Selbstverständlich liegt es auch im Rahmen der Erfindung, die Veresterung durch direkte Umsetzung der 2,2-Dimethylcyclopropancarbonsäure mit dem Mandelsäuremethylester in Gegenwart eines Katalysators, wie beispielsweise Dicyclohexylcarbodiimid oder 1,1'-Carbonyldiimidazol durchzuführen. Solche Veresterungsmethoden sind dem Fachmann bekannt und beispielsweise in "Methoden der organischen Chemie" (Houben-Weyl), 4.Auflage, Bd. E5, S.659ff und Bd.VIII, S.516ff beschrieben.

Die nach der Veresterung vorliegenden diastereomeren Ester werden fraktionierend kristallisiert, als Lösungsmittel wird vorzugsweise ein Alkan verwendet. Besonders bevorzugt als Lösungsmittel ist n-Hexan. Aus n-Hexan kristallisiert zuerst das Diastereomere mit gleicher absoluter Konfiguration an beiden Asymmetriezentren aus.

Zur Gewinnung von (S)-(+)-2,2-Dimethylcyclopropancarbonsäure in optisch reiner Form verwendet man daher vorteilhaft den (S)-(+)-Mandelsäuremethylester, entsprechend für die (R)-Säure den (R)-Ester. Ein Vorteil des erfindungsgemässen Verfahrens liegt darin, dass beide Enantiomere der Mandelsäure und damit auch deren Ester gut zugänglich sind.

Nach der Isolierung des gewünschten diastereomeren Esters wird dieser hydrolysiert. Die Hydrolyse wird vorzugsweise nach der üblichen Methode mit wässriger Alkalihydroxidlösung durchgeführt, wobei beide Estergruppen im Molekül hydrolysiert werden. Anschliessend werden durch Zusatz einer starken Säure, beispielsweise Salzsäure, die 2,2-Dimethylcyclopropancarbonsäure und die Mandelsäure, die nach der Hydrolyse zunächst als Anionen vorliegen, in Freiheit gesetzt. Die Abtrennung der optisch reinen 2,2-Dimethylcyclopropancarbonsäure erfolgt vorzugsweise durch Extraktion mit einem unpolaren Lösungsmittel.

Besonders bevorzugt als Extraktionsmittel sind geradkettige, verzweigte oder cyclische Alkane mit 5 bis 10 C-Atomen. Ganz besonders bevorzugt ist n-Hexan, in dem Mandelsäure praktisch unlöslich ist.

Die so erhaltene optisch reine 2,2-Dimethylcyclopropancarbonsäure kann auf bekannte Weise weiterverarbeitet werden, beispielsweise durch Überführung in das Säurechlorid und weiter in das Amid.

Zur Verwertung der Mutterlauge der Kristallisation, in der das leichter lösliche Diastereomere angereichert ist, wird diese vorteilhaft ebenfalls einer Hydrolyse unterworfen.

Das dadurch erhaltene Enantiomerengemisch wird zweckmässig wieder in das Gemisch der Säurechloride übergeführt, das auf an sich bekannte Weise durch Erhitzen auf 100 bis 200°C racemisiert werden kann. Das so erhältliche racemische Säurechlorid kann wieder dem Ausgangsmaterial des erfindungsgemässen Verfahrens zugesetzt werden, so dass weder das unerwünschte Enantiomere entsorgt werden muss noch nennenswerte Verluste auftreten.

Die nachfolgenden Beispiele verdeutlichen die Durchführung des erfindungsgemässen Verfahrens.

## Beispiel 1

### (RS)-2,2-Dimethylcyclopropancarbonsäurechlorid

In 50 ml n-Hexan wurden 52,8 g (RS)-2,2-Dimethylcyclopropancarbonsäure und 0,25 g N,N-Dimethylformamid gelöst und unter Rückfluss tropfenweise mit 104,0 g Thionylchlorid in 100 ml n-Hexan versetzt. Nach weiteren 2 Stunden Rühren unter Rückfluss wurde das Lösungsmittel abdestilliert und der Rückstand bei 200 mbar und 100°C (Badtemperatur) rasch destilliert. Ausbeute: 55,0 g

## Beispiel 2

### (S,S)-α-(2,2-Dimethylcyclopropancarbonyloxy)-phenylessigsäuremethylester

In 70 ml Dichlormethan wurden 14,0 g (RS)-2,2-Dimethylcyclopropancarbonsäurechlorid gelöst, auf 0°C abgekühlt und so schnell wie möglich mit 8,1 g Pyridin versetzt.

Anschliessend wurde zu dieser Mischung bei 0 bis 5°C innerhalb von 10 Minuten eine Lösung von 17,0 g (S)-(+)-Mandelsäuremethylester ($[\alpha]_D^{20}$ = +146,5° (c=1, MeOH)) in 35 ml Dichlormethan getropft.

Das Reaktionsgemisch wurde noch 2 Stunden bei Raumtemperatur gerührt und anschliessend nacheinander mit Wasser, verdünnter Salzsäure und nochmals mit Wasser gewaschen. Die organische Phase wurde über Natriumsulfat getrocknet und eingedampft. Das so erhaltene Rohprodukt (26,0 g) wurde in 7,0 ml n-Hexan bei Raumtemperatur aufgeschlämmt. Der kristalline Rückstand wurde abfiltriert, getrocknet und dreimal aus je 40 ml n-Hexan heiss umkristallisiert.

Ausbeute: 7,6 g (S,S)-α-2,2-(Dimethylcyclopropancarbonyloxy)phenylessigsäuremethylester
Schmp:
80-82°C, farblose Kristalle
$[\alpha]_D^{20}$ :
+158,0° (c=1, $CHCl_3$)
$^1$H-NMR (300 MHz, $C_6D_6$) :
δ 7,43-7,52 (m,2H), 6,97-7,12 (m,3H), 6,11 (s,1H), 3,19 (s,3H), 1,55-1,60 (m,1H), 1,39 (s,3H), 1,19-1,23 (m,1H), 0,86 (s,3H), 0,55-0,60 (m,1H)

## Beispiel 3

### (S)-(+)-2,2-Dimethylcyclopropancarbonsäure

18,4 g (S,S)-α-(2,2-Dimethylcyclopropancarbonyloxy)phenylessigsäuremethylester (hergestellt nach Beispiel 2) wurden mit einer Lösung von 20,6 g Kaliumhydroxid (85%ig) in 235 ml Wasser versetzt und 6 Stunden bei 80°C gerührt, wobei sich eine klare Lösung bildete.

Anschliessend wurde das Reaktionsgemisch auf Raumtemperatur abgekühlt und mit verdünnter Salzsäure auf pH 1 angesäuert. Die wässrige Lösung wurde viermal mit je 100 ml n-Hexan extrahiert.

Die vereinigten organischen Phasen wurden über Natriumsulfat getrocknet und filtriert. Nach Abdestillieren des Lösungsmittels wurde (S)-(+)-2,2-Dimethylcyclopropancarbonsäure in einer Reinheit (GC) von 99,0% erhalten.

Ausbeute: 7,7 g

$[\alpha]_D^{20}$ : +146° (ohne Lösungsmittel) entsprechend einer optischen Reinheit (ee-Wert) ≧ 98%.

### Beispiel 4

### Racemisierung von 2,2-Dimethylcyclopropancarbonsäure

Aus den bei der Kristallisation von (S,S)-α-(2,2-Dimethylcyclopropancarbonyloxy)-phenylessigsäuremethylester gemäss Beispiel 2 anfallenden Mutterlaugen wurde das Lösungsmittel abdestilliert und der Rückstand analog zu Beispiel 3 alkalisch hydrolysiert und aufgearbeitet. Man erhielt 11,4 g 2,2-Dimethylcyclopropancarbonsäure (-$[\alpha]_D^{20}$ = -51,8° (c = 1, CHCl$_3$)), bestehend aus 69% (R)-(-)-Form und 31% (S)-(+)-Form. Das Enantiomerengemisch wurde mit 12,0 g Hexan verdünnt, auf 75°C erhitzt und innerhalb von 30 Minuten tropfenweise mit einer Mischung aus 17,9 g Thionylchlorid und 5,0 g Hexan versetzt und noch 2,5 Stunden am Rückfluss gekocht.

Das Lösungsmittel und das überschüssige Thionylchlorid wurden abdestilliert und der Rückstand unter Rühren 2 Stunden auf 135°C erhitzt.

Nach dem Abkühlen auf Raumtemperatur wurde das Säurechloridgemisch mit verdünnter Natronlauge hydrolysiert und die entstandene wässrige Lösung zweimal mit je 10 g Toluol extrahiert. Die organische Phase wurde verworfen, die wässrige Phase mit konzentrierter Salzsäure angesäuert und fünfmal mit je 40 g Hexan extrahiert.

Aus den Hexanphasen erhielt man durch Abdestillieren des Lösungsmittels 11,4 g rohe 2,2-Dimethylcyclopropancarbonsäure, die im Wasserstrahlvakuum destilliert wurde.

Ausbeute:    9,4 g (83%) farblose, übelriechende Flüssigkeit bestehend aus 48,5% (S)-(+)-Form und 51,5% (R)-(-)-Form.

## Patentansprüche

1. Verfahren zur Racematspaltung von 2,2-Dimethylcyclopropancarbonsäure durch Veresterung mit einer optisch aktiven Hydroxyverbindung, fraktionierende Kristallisation der gebildeten diastereomeren Ester und anschliessende Hydrolyse, dadurch gekennzeichnet, dass als optisch aktive Hydroxyverbindung Mandelsäuremethylester eingesetzt wird.

2. Verfahren nach Patentanspruch 1, dadurch gekennzeichnet, dass die fraktionierende Kristallisation der diastereomeren Ester mit einem Alkan als Lösungsmittel durchgeführt wird.

3. Verfahren nach Patentanspruch 2, dadurch gekennzeichnet, dass als Alkan n-Hexan eingesetzt wird.

4. Verfahren nach einem oder mehreren der Patentansprüche 1 bis 3, dadurch gekennzeichnet, dass die Veresterung der 2,2-Dimethylcyclopropancarbonsäure über das entsprechende racemische Säurechlorid in Gegenwart einer Hilfsbase erfolgt.

5. Verfahren nach Patentanspruch 4, dadurch gekennzeichnet, dass das racemische Säurechlorid durch Umsetzung der 2,2-Dimethylcyclopropancarbonsäure mit Thionylchlorid hergestellt wird.

6. Verfahren nach Patentanspruch 4 oder 5, dadurch gekennzeichnet, dass das racemische Säurechlorid unter Verwendung des aus der Mutterlauge der Kristallisation durch Hydrolyse zurückgewonnenen Enantiomerengemisches von 2,2-Dimethylcyclopropancarbonsäure hergestellt und durch Erhitzen auf 100 bis 200°C racemisiert wird.

7. Verfahren nach einem oder mehreren der Patentansprüche 1 bis 6, dadurch gekennzeichnet, dass die Hydrolyse der Ester der 2,2-Dimethylcyclopropancarbonsäure mit einem wässrigen Alkalihydroxid durchgeführt wird.

8. Verfahren nach einem oder mehreren der Patentansprüche 1 bis 7, dadurch gekennzeichnet, dass die 2,2-Dimethylcyclopropancarbonsäure durch Ansäuern und Extraktion mit n-Hexan aus dem Hydrolysegemisch isoliert wird.

9. α-(2,2-Dimethylcyclopropancarbonyloxy)-phenylessigsäuremethylester.

## Claims

1. A process for the resolution of racemic 2,2-dimethylcyclopropane carboxylic acid by esterification with an optically active hydroxy compound, fractionating crystallization of the diastereomeric esters thus formed and subsequent hydrolysis, characterized by using amygdalic acid methyl ester as an optically active hydroxy compound.

2. The process according to Claim 1 , characterized in that fractionating crystallization of the diastereomeric esters is conducted with an alkane as solvent.

3. The process according to Claim 2, characterized by using n-hexane as an alkane.

4. The process according to one or more of Claims 1 to 3, characterized in that esterification of the 2,2-dimethylcyclopropane carboxylic acid via the respective racemic acid chloride takes place in the presence of an auxiliary base.

5. The process according to Claim 4, characterized in that the racemic acid chloride is prepared by reaction of 2,2-dimethylcyclopropane carboxylic acid with thionyl chloride.

6. The process according to Claim 4 or 5, characterized in that the racemic acid chloride is prepared under use of the mixture of enantiomers of 2,2-dimethyl cyclopropane carboxylic acid recovered through hydrolysis from the mother liquor of crystallization and is racemized by heating to 100 to 200 °C.

7. The process according to one or more of Claims 1 to 6, characterized in that hydrolysis of the esters of 2,2-dimethyl cyclopropane carboxylic acid is conducted with an aqueous alkali hydroxide.

8. The process according to one or more of Claims 1 to 7, characterized in that the 2,2-dimethyl cyclopropane carboxylic acid is isolated from the hydrolytic mixture by acidification and extraction with n-hexane.

9. Alpha-(2,2-dimethyl cyclopropane carbonyloxy) phenyl acetic acid methyl ester.

## Revendications

1. Procédé pour la dissociation ou dédoublement racémique de 2,2-diméthylcyclopropane-carboxylique par estérification avec un composé hydroxy actif optiquement, cristallisation fractionnante de l'ester diastéréoisomérique formé et hydrolyse consécutive, caractérisé en ce qu'en tant que composé hydroxy optiquement actif on met en oeuvre un méthylester de l'acide mandélique ou phénylglycolique.

2. Procédé selon la revendication 1, caractérisé en ce que la cristallisation fractionnante de l'ester diastéréoisomérique est conduite avec un alcane comme solvant.

3. Procédé selon la revendication 2, caractérisé en ce qu'en tant qu'alcane on met en oeuvre un n-hexane.

4. Procédé selon une ou plusieurs des revendications 1 à 3, caractérisé en ce que l'estérification de l'acide 2,2-diméthylcyclopropanecarboxylique s'effectue par le biais du chlorure d'acide racémique correspondant en présence d'une base auxiliaire.

5. Procédé selon la revendication 4, caractérisé en ce que le chlorure d'acide racémique est préparé par mise en réaction de l'acide 2,2-dyméthylcyclopropanecarboxylique avec un chlorure de thionyle.

6. Procédé selon la revendication 4 ou 5, caractérisé en ce que le chlorure de l'acide racémique est préparé en utilisant le mélange énantiomère de l'acide 2,2-dyméthylcyclopropanecarboxylique récupéré de la lessive mère de la cristallisation par hydrolyse et racémisé par chauffage à 100 jusqu'à 200 °C.

7. Procédé selon une ou plusieurs des revendications 1 à 6, caractérisé en ce que l'hydrolyse de l'ester de l'acide 2,2-dyméthylcyclopropanecarboxylique est conduite avec un hydroxyde alcalin aqueux.

8. Procédé selon une ou plusieurs des revendications 1 à 7, caractérisé en que l'acide 2,2-diméthylcyclopropanecarboxylique est isolé par acidification et extraction avec du n-hexane à partir du mélange d'hydrolyse.

9. Méthylester de l'acide α-(2,2-diméthylcyclopropanecarbonyloxy) phénylacétique.